# EUROPÄISCHE PATENTSCHRIFT

(11) **EP 0 808 633 B1**
(45) Veröffentlichungstag und Bekanntmachung des Hinweises auf die Patenterteilung: **23.10.2002**
(21) Anmeldenummer: 97108082.5
(22) Anmeldetag: 17.05.1997
(51) Int. Cl.: A61M 1/16

(54) **Vorrichtung zum Entfernen von Gasen aus Flüssigkeiten**
Liquid degassing device
Dispositif de dégazage d'un liquide

(30) Priorität: 22.05.1996 DE 19620591
(43) Veröffentlichungstag der Anmeldung: 26.11.1997
(73) Patentinhaber: Fresenius Medical Care Deutschland GmbH, 61352 Bad Homburg v.d.H. (DE)
(72) Erfinder: Wamsiedler, Ralf, 97453 Schonungen (DE)
(74) Vertreter: Luderschmidt, Schüler & Partner GbR

(56) Entgegenhaltungen:
- US-A- 3 827 561
- US-A- 3 920 556
- US-A- 4 344 777
- US-A- 4 368 118

## Beschreibung

Die Erfindung betrifft eine Vorrichtung zum Entfernen von Gasen aus Flüssigkeiten, insbesondere aus einer Dialysierflüssigkeit, gemäß dem Oberbegriff des Patentanspruchs 1.

Eine derartige Vorrichtung ist beispielweise aus der US-A-4 368 118 bekannt.

Beim Aufbau eines extrakorporalen Blutkreislaufs sowie bei der Herstellung von Dialysierflüssigkeiten für die Dialyse ist eine Vielzahl von Einrichtungen zur Abscheidung von Luft in Form von Blasenfallen oder Luftabzugseinrichtungen bekannt, die die Aufgabe haben, die in der Flüssigkeit vorhandenen, zum Teil gelösten Gase von der Flüssigkeit wirksam zu trennen.

Die bei der Dialyse zur Anwendung kommenden Dialysierflüssigkeiten setzen bei Unterdruck Gase frei, wobei Luftabscheider zum Einsatz kommen, die einen oberen Einlaß aufweisen, der üblicherweise seitlich angeordnet ist und der einlaufenden Flüssigkeit einen spiralförmigen Weg aufprägen soll. Dabei sollen die Luftblasen durch einen oben liegenden hydrophoben Filter abgeschieden werden. Eine derartige Anordnung ist beispielsweise aus der DE 32 15 003 bekannt. In der Praxis wurde jedoch festgestellt, daß durch den entstehenden Sog die abgeschiedene Luft zum Teil in den Auslauf mitgerissen wird, so daß weitere Vorkehrungen getroffen werden mußten, um die Abscheidung zu verbessern. So wurden beispielsweise Abscheidehilfen in Form kleiner Bleche in den Abzugsraum eingebracht. Des weiteren wurden die Luftabscheider häufig sehr lang gebaut, um die Abscheidungsfläche möglichst groß zu gestalten.

Aus US-A-4,061,031 ist eine Vorrichtung bekannt, die sowohl als Durchflußmesser als auch als Luftabscheider Verwendung finden kann. Die bekannte Vorrichtung weist einen Behälter auf, der durch eine Trennwand in zwei Kammern unterteilt ist, von denen die eine Kammer mit der Einlaßöffnung und die andere Kammer mit der Auslaßöffnung versehen ist. Die Trennwand weist einen Durchbruch auf und erstreckt sich vom Boden des Behälters bis nahe an die Behälterdecke unter Ausbildung eines spaltförmigen Zwischenraumes zwischen der Einlaßkammer und der Auslaßkammer sowie der Behälterdecke.

Die durchbrochene Trennwand wird durch zwei versetzt zueinander angeordnete, plattenförmige Elemente gebildet, wobei die Öffnungen der Ein- und Auslaßkammern sich am Boden des Behälters befinden. Da der Durchbruch einen geringeren Querschnitt als die Einlaßöffnung aufweist, bilden sich in den Kammern Flüssigkeitssäulen unterschiedlicher Höhen aus, wodurch es möglich ist, den Durchfluß zu messen. Dabei ist die Differenz zwischen den beiden Flüßigkeitsäulen ein Maß für die Höhe des Durchflusses.

Bei der bekannten Vorrichtung dienen beide Kammern als Blasenfalle. Die in der Flüssigkeit befindlichen Luftblasen steigen in den Kammern nach oben und verbleiben als Gas über dem Flüssigkeitsspiegel. Eine aktive Abscheidung ist bei der bekannten Vorrichtung nicht möglich. Damit die Flüssigkeit nicht über den oberen Rand der Trennwand in die Auslaßkammer strömt, weist die Einlaßkammer eine langgestreckte Form auf, was zu einer verhältnismäßig großen Bauhöhe führt.

Die US 4,368,118 A beschreibt eine Vorrichtung zum Entfernen von Gasen aus Flüssigkeiten mit einem Behälter, der durch eine Trennwand in zwei Kammern unterteilt ist. Die beiden Kammern stehen unterhalb der Behälterdecke über einen spaltförmigen Zwischenraum in Strömungsverbindung. Während die Flüssigkeit aus der einen über den Zwischenraum in die andere Kammer strömt, werden Gasblasen abgeschieden, die sich an der Behälterdecke sammeln. Diese Gasblasen können dann über eine an der Behälterdecke vorgesehene Ventilanordnung abgelassen werden.

Die US 4,344,777 beschreibt eine ähnliche Vorrichtung zum Entfernen von Gasen aus Flüssigkeiten. Auch bei dieser Vorrichtung sammeln sich die Gasblasen unterhalb der Behälterdecke, von wo sie über eine Ventilanordnung abgelassen werden können.

Der Erfindung liegt die Aufgabe zugrunde, eine Vorrichtung zum Entfernen von Gasen aus Flüssigkeiten zu schaffen, mit der sich bei kompakter Bauweise und einfacher Konstruktion eine hohe Abscheiderate erzielen läßt.

Die Lösung der Aufgabe erfolgt erfindungsgemäß mit den Merkmalen des Patentanspruchs 1.

Vorteilhafte Weiterbildungen der Erfindung sind Gegenstand der abhängigen Ansprüche.

Bei der erfindungsgemäßen Vorrichtung ist die Trennwand als durchgehender Körper ausgebildet, so daß die erste Öffnung und die zweite Öffnung nur über den spaltförmigen Zwischenraum in Strömungsverbindung stehen. Ferner ist an der Behälterdecke eine Entlüftungsöffnung vorgesehen, die durch eine Entlüftungseinheit verschlossen ist, die zwar Gas, aber keine Flüssigkeit durchläßt. Während des Betriebs ist der Behälter, d.h. die beiden Kammern und der spaltförmige Zwischenraum, vollständig mit Flüssigkeit gefüllt. Die zu entgasende Flüssigkeit strömt aus der einen Kammer über den spaltförmigen Zwischenraum unterhalb der Behälterdecke in die andere Kammer. An der Trennwand findet eine Umkehr der Flußrichtung statt, wobei die in der Flüssigkeit enthaltenen Gase durch die an der Behälterdecke vorgesehene Entlüftungsöffnung austreten können.

Bei der erfindungsgemäßen Vorrichtung wird die Entlüftungsöffnung von einer hydrophoben Membran verschlossen, so daß die zu entgasende Flüssigkeit gegen die Membran gedrückt wird. Die hydrophobe Membran sollte für die Flüssigkeit eine möglichst große Angriffsfläche bilden und sich vorzugsweise nahezu über die gesamte Querschnittsfläche des spaltförmigen Zwischenraums erstrecken.

Eine besonders hohe Abscheiderate bei kompakter Bauweise der Vorrichtung wird insbesondere dann erreicht, wenn die Trennwand in Form eines Hohlkörpers ausgebildet ist, der unter Ausbildung der ersten und zweiten Kammer innerhalb des Behälters derart angeordnet ist, daß die innenliegende erste Kammer einen geringeren Querschitt als die außen liegende zweite Kammer aufweist. Bei dieser Anordnung bildet die erste Kammer die Zuflußkammer und die zweite Kammer bildet die Abflußkammer. Da die Zuflußkammer einen geringeren Querschnitt als die Abflußkammer aufweist, wird die Fließgeschwindigkeit in der ersten Kammer und damit der statische Druck auf die Entlüftungsöffnung erhöht. Andererseits wird der dynamische Druck an der Entlüftungsöffnung klein gehalten, so daß trotz der in der ersten Kammer erhöhten Strömungsgeschwindigkeit praktisch keine Luft mitgerissen wird.

Die Zuführungsöffnung zur ersten Kammer ist in einer bevorzugten Ausführungsform so ausgestaltet, daß die zugeführte Flüssigkeit horizontal einströmen kann, wobei dieser horizontalen Strömungskomponente vorteilhafterweise eine tangentiale Stömungskomponente aufgeprägt wird. Dies hat zur Folge, daß die einströmende Flüssigkeit im wesentlichen spiralförmig nach oben befördert wird, wodurch ebenfalls die Luftabscheidung verbessert wird. Das zulaufende Fluidgemisch wird also in eine Rotationsbewegung innerhalb des erfindungsgemäßen Luftabscheiders versetzt, wodurch Gas in Richtung der gemeinsamen Achse zwangsweise gefördert wird.

Bei einer weiteren bevorzugten Ausführungsform ist der Boden des rohrförmigen Innenteils der ersten Kammer gegenüber dem Boden des Außenteils der zweiten Kammer nach unten verlängert und ragt demgemäß nach unten vor. Bei dieser Ausführungsform ist die Zulauföffnung an der Rohrseitenwand benachbart zum Boden angeordnet, wobei der mit der ersten Öffnung in Strömungsverbindung stehende Zuführstutzen vorteilhafterweise tangential zur Rohrwand in die erste Öffnung mündet.

In ähnlicher Weise kann der Auslaufstutzen aus der zweiten Öffnung ebenfalls horizontal benachbart zum Boden angeordnet sein und gegebenenfalls tangential zur Behälterwand in die zweite Öffnung münden.

Besonders bevorzugt ist die Ausführungsform, bei der die horizontalen Wände der ersten Kammer und der zweiten Kammer kreisringförmig ausgebildet sind und somit eine Rohr-in-Rohr-Anordnung bilden, so daß zwischen der ersten Kammer und der zweiten Kammer ein kreisförmiger Ringraum ausgebildet ist.

Im folgenden wird ein Ausführungsbeispiel der Erfindung unter Bezugnahme auf die Zeichnungen näher erlaäutert.

Es zeigen:
- Fig. 1: einen Längsschnitt durch eine erste Ausführungsform der Erfindung und
- Fig. 2: eine Ansicht der Ausführungsform von Figur 1 von unten.

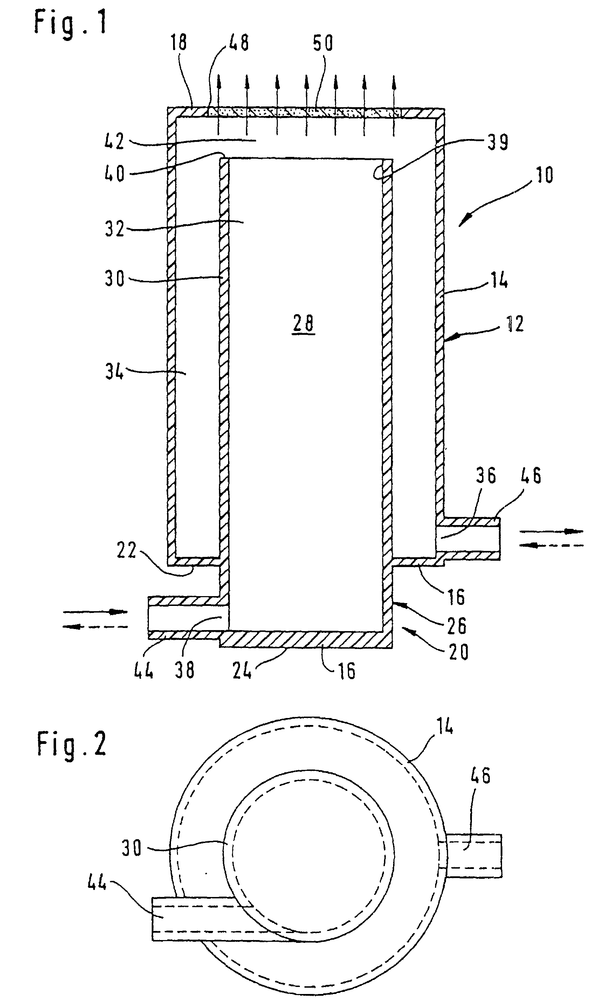

In Fig. 1 is mit 10 eine Vorrichtung zum Entfernen von Gasen aus medizinischen Flüssigkeiten, insbesondere zum Entfernen von Luft aus einer Dialysierflüssigkeit dargestellt, die einen Behälter 12 aufweist. Dieser Behälter 12 verfügt über eine im wesentlichen vertikal angeordnete Behälterwand 14, die gemäß der in Fig. 1 gezeigten Ausführungsform im wesentlichen kreisrund ausgeführt ist. Der Behälter 12 wird auf seiner Unterseite durch einen Behälterboden 16 und auf seiner Oberseite durch eine Behälterdecke 18 begrenzt.

Gemäß der in Fig. 1 gezeigten Ausführungsform ist der Behälterboden 16 unter Bildung einer Stufe 20 abgesetzt, wodurch der Behälterboden in einen äußeren ringförmigen Bereich 22 und einen inneren im wesentlichen kreisförmigen Bereich 24 geteilt ist. Demzufolge bildet der gestufte Bereich 20 einen im wesentlichen zylinderförmigen Bereich 26.

Der Behälter 12 weist einen Innenraum 28 auf, der durch eine ringförmig geschlossene Wand 30 in eine erste Kammer 32, die sich innerhalb der ringförmigen Wand 30 befindet, und eine zweite Kammer 34 geteilt ist, die eine im wesentlichen ringförmige Struktur aufweist, die ringförmige Wand 30 umgibt und durch die Behälterwand 14 umgrenzt ist.

Die ringförmige Wand 30 ist auf dem Behälterboden 16 befestigt und gemäß der in Fig. 1 gezeigten Ausführungsform so ausgebildet, daß sie am ringförmigen Bodenbereich 22 in den zylinderförmigen Bereich 26 übergeht und mit diesem Bereich zusammenfällt. Andererseits kann jedoch aber auch der gesamte Boden 16 eben ausgebildet sein, so daß die ringförmige Wand 30 auf diesem Boden steht und mit diesem Boden befestigt ist.

Im Bereich des Bodens 16 ist im Bereich der ersten Kammer 32 eine erste Öffnung 38 vorgesehen, die vorteilhafterweise als Zuführungsöffnung eingesetzt wird. Diese erste Öffnung 38 ist gemäß der in Fig. 1 gezeigten Ausführungsform im nach unten überstehenden zylinderförmigen Bereich 26 angeordnet.

Das dem Boden 16 gegenüberliegende Ende der ringförmigen Wand 30 ist in die Nähe der Behälterdecke 18 geführt und mündet dort in eine Rohröffnung 39, die durch einen umlaufenden Rohrrand 40 begrenzt wird, der ein Überlaufwehr für eine zugeführte Flüssigkeit darstellt. Zwischen dem Rohrrand 40 und der Behälterdecke 18 ist ein spaltförmiger Strömungsraum 42 vorgesehen, der eine Strömungsverbindung zwischen der ersten Kammer 32 und der zweiten Kammer 34 über den Rohrrand 40 hinweg schafft.

Die Querschnittsform des Behälters 12 sowie seiner Teile 14 und 30 ist im wesentlichen nicht kritisch, vorzugsweise jedoch kreisförmig, wobei die beiden Teilkreise der Außenwand 14 und der Wand 30 vorzugsweise einen gemeinsamen Kreismittelpunkt haben.

Vorteilhafterweise ist die innenliegende erste Kammer 32 als Zuflußkammer ausgestaltet, während die konzentrisch außenliegende zweite Kammer 34 als Abflußkammer dient. Dabei weist die Abflußkammer vorteilhafterweise einen größeren Querschnitt auf als die Zuflußkammer, so daß die Flüssigkeit über den Rohrrand 40 hinweg im wesentlichen ungehindert fließen kann. In dem ganzen Behälter herrscht keine wesentliche Druckdifferenz.

Hinzuzufügen ist jedoch, daß auch eine andere Anströmung des Behälters möglich ist, beispielsweise auch eine Strömung von außen nach innen, obwohl dies nicht bevorzugt ist.

Gemäß der Ausführungsform von Fig. 1 und 2 ist an der ersten Kammer 32 im Bereich der ersten Rohröffnung 38 ein erster Rohrstutzen 44 angeordnet. Dabei mündet die Rohrlängsachse des Rohrstutzens 44 - wie aus Fig. 2 ersichtlich ist - vorteilhafterweise tangential in die Kreisform der ersten Kammer 32 ein und verläuft im übrigen in der Gebrauchslage im wesentlichen horizontal.

Weiterhin ist an der zweiten Kammer 34 im Bereich der zweiten Öffnung 36 ein zweiter Rohrstutzen 46 angeordnet, der als Abflußstutzen dient, wobei dessen Achse vorteilhafterweise ebenfalls in Gebrauchslage horizontal angeordnet ist.

Die an den Rohrstutzen 44 und 46 in Fig. 1 oder 3 gezeigten Pfeile zeigen dabei die jeweiligen Zu- und Abflußrichtungen an.

Des weiteren weist die Behälterdecke 18 eine Entlüftungsöffnung 48 auf, die mit einer Entlüftungseinheit verschlossen ist. Bei der in Fig. 1 gezeigten Ausführungsform ist die Entlüftungseinheit ein mikroporöser hydrophober Filter 50, der das abgetrennte Gas, nicht jedoch die wässrige Flüssigkeit durchläßt. Soweit der hydrophobe Filter betroffen ist, wird auf die Offenbarung der DE 32 15 003 Bezug genommen.

Der Behälter 12 wird üblicherweise aus einem Kunststoffmaterial hergestellt und dient zur Abscheidung von Luft bei der Herstellung von z.B. Dialysierflüssigkeit als Primärluftabscheider. Dabei wird aus der frisch zubereiteten Flüssigkeit zunächst in einer Unterdruckstrecke die physikalisch in der Flüssigkeit gelöste Luft freigesetzt. Das Flüssigkeits-/Luft-Gemisch wird dem Rohrstutzen 44 zugeführt, wobei aufgrund der tangentialen Einleitung sich das Gemisch schraubenförmig nach oben bewegt und dadurch die Luft in Richtung Körperachse unter Bildung von kleinen Luftblasen zwangsweise von der Flüssigkeit getrennt wird.

Im Bereich der hydrophoben Membran 50 bzw. des Ladedruckventils 56 erfolgt dann die Abscheidung von Luft.

Ein großer Vorteil der erfindungsgemäßen Vorrichtung zum Entfernen von Gasen ist die einfache kontinuierliche, sehr effiziente Luftabscheidung, insbesondere bei der single-pass-Dialyse. Ein weiterer Vorteil ist es, daß der Behälter der Vorrichtung ständig mit Flüssigkeit gefüllt ist, so daß hierdurch eine sehr gute Desinfizier- bzw. Freispülbarkeit gegeben ist. Schließlich können die Abmessungen der erfindungsgemäßen Vorrichtung aufgrund ihrer guten Trenneigenschaften gegenüber den derzeit eingesetzten Luftabscheidern etwa um die Hälfte verringert werden.

## Patentansprüche

1. Vorrichtung zum Entfernen von Gasen aus Flüssigkeiten, insbesondere aus einer Dialysierflüssigkeit, mit einem Behälter (12), der in der Gebrauchslage eine unten liegende erste Öffnung (38) und eine unten liegende zweite Öffnung (36) aufweist, und einer innerhalb des Behälters (12) angeordneten Trennwand (30), die den Behälter in eine erste Kammer (32), die die erste Öffnung (38) aufweist, und eine zweite Kammer (34) teilt, die die zweite Öffnung (36) aufweist, und die sich vom Behälterboden (24) bis nahe zur Behälterdecke (18) unter Bildung eines spaltförmigen Zwischenraumes (42) zwischen der ersten Kammer (32) und der zweiten Kammer (34) sowie der Behälterdecke (18) erstreckt, wobei die Trennwand (30) als durchgehender Körper ausgebildet ist, so daß die erste Öffnung (38) und die zweite Öffnung (36) nur über den spaltförmigen Zwischenraum (42) in Strömungsverbindung stehen, **dadurch gekennzeichnet, daß** die Behälterdecke (18) eine mit einer hydrophoben Membran (50), die Gas, jedoch keine Flüssigkeit durchläßt, verschlossene Entlüftungsöffnung (48) aufweist, so daß die Flüssigkeit unter Abscheidung der Gasblasen durch den Zwischenraum (42) strömt, während der Behälter (12) vollständig mit Flüssigkeit gefüllt ist.

2. Vorrichtung nach Anspruch 1, **dadurch gekennzeichnet, daß** die Trennwand (30) in Form eines Hohlkörpers ausgebildet ist, der sich unter Ausbildung der ersten Kammer (32) und der zweiten Kammer (34) vom Behälterboden (24) bis nahe zur Behälterdecke (18) erstreckt.

3. Vorrichtung nach Anspruch 2, **dadurch gekennzeichnet, daß** die innenliegende erste Kammer (32) einen geringeren Querschnitt als die außenliegende zweite Kammer aufweist, wobei die erste Kammer die Zuflußkammer und die zweite Kammer die Abflußkammer bildet.

4. Vorrichtung nach Anspruch 2 oder 3, **dadurch gekennzeichnet, daß** der die Trennwand (30) bildende Hohlkörper im wesentlichen zylinderförmig ausgebildet ist.

5. Vorrichtung nach Anspruch 4, **dadurch gekennzeichnet, daß** die die außenliegende zweite Kammer (34) begrenzende Behälterwand (14) im wesentlichen zylinderförmig ausgebildet ist und den die Trennwand (30) bildenden zylinderförmigen Körper konzentrisch umgibt.

6. Vorrichtung nach einem der Ansprüche 1 bis 5, **dadurch gekennzeichnet, daß** der in die erste Öffnung (38) mündende erste Rohrstutzen (44) und der in die zweite Öffnung (36) mündende zweite Rohrstutzen (46) in Gebrauchslage im wesentlichen horizontal angeordnet sind.

7. Vorrichtung nach Anspruch 6, **dadurch gekennzeichnet, daß** der erste Rohrstutzen (47) derart angeordnet ist, daß die in die erste Kammer (32) strömende Flüssigkeit in eine Rotationsbewegung versetzt wird.

## Claims

1. Device for the removal of gases from liquids, in particular a dialysing liquid, with a container (12) which when standing in the operating position exhibits a low-level first opening (38) and a low-level second opening (36) together with a separating wall (30) arranged within the container (12) which divides the container into a first chamber (32) which exhibits the first opening (38) and a second chamber (34) which exhibits the second opening (36) and which extends from the base of the container (24) almost up to the top of the container (18) forming a narrow gap (42) between the first chamber (32) and the second chamber (34) as well as the top of the container (18) whereby the separating wall (30) has the form of a continuous body such that the first opening (38) and the second opening (36) are in flow contact with one another only by reason of the narrow gap (42), **characterised in that** the top of the container (18) exhibits a hydrophobic membrane (50) which permits gas to pass through but not a liquid and constitutes a sealed ventilation opening (48) so that the liquid being freed from gas bubbles flows through the gap (42) while the container (12) is completely filled with liquid,

2. Device in accordance with claim 1, **characterised in that** the separating wall (30) has the form of a hollow body which extends from the base of the container (24) almost to the top of the container (18) and forms the first chamber (32) and the second chamber (34).

3. Device in accordance with claim 2, **characterised in that** the inner first chamber (32) exhibits a smaller cross-sectional area than the outer second chamber, whereby the first chamber constitutes the inflow chamber and the second chamber the out-flow chamber.

4. Device in accordance with claims 2 or 3, **characterised in that** the hollow body forming the separating wall (30) is essentially shaped as a cylinder.

5. Device in accordance with claim 4, **characterised in that** the container wall (14) limiting the outer second chamber (34) is essentially shaped as a cylinder and surrounds the cylindrical body forming the separating wall (30) in a concentric manner.

6. Device in accordance with one of claims 1 to 5, **characterised in that** the first pipe socket (44) running into the first opening (38) and the second pipe socket (46) running into the second opening (36) essentially lie in a horizontal position when the device is in its operating position.

7. Device in accordance with claim 6, **characterised in that** the first pipe socket (47) is arranged in such a manner that the liquid flowing into the first chamber (32) is given a rotational movement.

## Revendications

1. Dispositif pour éliminer les gaz de liquides, en particulier d'un liquide de dialyse, comprenant un récipient (12) qui présente, dans la position d'utilisation, une première ouverture (38) disposée dans la partie inférieure et une deuxième ouverture (36) disposée dans la partie inférieure, et une paroi de séparation (30) disposée à l'intérieur du récipient (12), ladite paroi subdivisant le récipient en une première chambre (32) qui présente la première ouverture (38) et en une deuxième chambre (34) qui présente la deuxième ouverture (36) et s'étendant depuis le fond du récipient (24) jusqu'à un endroit situé à proximité du recouvrement de récipient (18) en formant un espace intermédiaire (42) en forme de fente entre la première chambre (32) et la deuxième chambre (34) et le recouvrement de récipient (18), par lequel la paroi de séparation (30) est réalisée sous la forme d'un corps continu de telle sorte que la première ouverture (38) et la deuxième ouverture (36) n'entrent en liaison d'écoulement que via l'espace intermédiaire (42) en forme de fente, **caractérisé en ce que** le recouvrement de récipient (18) présente une ouverture de dégazage (48) fermée par une membrane hydrophobe (50) qui laisse passer les gaz, mais pas les liquides, de telle sorte que les bulles de gaz se séparent du liquide lorsque ce dernier s'écoule à travers l'espace intermédiaire (42), tandis que le récipient (12) est rempli complètement avec du liquide.

2. Dispositif selon la revendication 1, **caractérisé en ce que** la paroi de séparation (30) est réalisée sous la forme d'un corps creux qui s'étend, en formant la première chambre (32) et la deuxième chambre (34), depuis le fond du récipient (24) jusqu'à un endroit proche du recouvrement de récipient (18).

3. Dispositif selon la revendication 2, **caractérisé en ce que** la première chambre interne (32) présente une section transversale inférieure à celle de la deuxième chambre externe, la première chambre formant la chambre d'amenée et la deuxième chambre formant la chambre d'évacuation.

4. Dispositif selon la revendication 2 ou 3, **caractérisé en ce que** le corps creux formant la paroi de séparation (30) est réalisé essentiellement en une forme cylindrique.

5. Dispositif selon la revendication 4, **caractérisé en ce que** la paroi de récipient (14) délimitant la deuxième chambre externe (34) est réalisée essentiellement en une forme cylindrique et entoure de manière concentrique le corps de forme cylindrique formant la paroi de séparation (30).

6. Dispositif selon l'une quelconque des revendications 1 à 5, **caractérisé en ce que** la première tubulure (44) débouchant dans la première ouverture (38) et la deuxième tubulure (46) débouchant dans la deuxième ouverture (36) sont disposées, dans la position d'utilisation, essentiellement à l'horizontale.

7. Dispositif selon la revendication 6, **caractérisé en ce que** la première tubulure (47) est agencée de telle sorte que le liquide qui s'écoule dans la première chambre (32) est soumis à un mouvement de rotation.
